(19) **Europäisches Patentamt**

**European Patent Office**

**Office européen des brevets**

(11) **EP 1 502 906 A1**

(12) **EUROPEAN PATENT APPLICATION**

(43) Date of publication:
**02.02.2005 Bulletin 2005/05**

(51) Int Cl.7: **C07C 17/358**

(21) Application number: **03102385.6**

(22) Date of filing: **31.07.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Solvay Solexis S.p.A.**
**20121 Milano (IT)**

(72) Inventors:
• **Cuzzato, Paolo**
**31050, Ponzano Veneto (IT)**

• **Basciutti, Paolo**
**30030, Favaro Veneto (IT)**
• **Bragante, Letanzio**
**35020, Due Carrare (IT)**

(74) Representative: **Jacques, Philippe et al**
**Solvay S.A.**
**Département de la Propriété Industrielle,**
**Rue de Ransbeek, 310**
**1120 Bruxelles (BE)**

(54) **Process for activation of AIF3 based catalysts and process for isomerising hydrochlorofluorocarbons**

(57) An activated $AlF_3$ based catalyst is produced by treating a crude $AlF_3$ for more than 5 hours with a gas stream at a temperature from 300°C to 450°C.

EP 1 502 906 A1

Printed by Jouve, 75001 PARIS (FR)

**Description**

The present invention concerns a process for producing an activated $AlF_3$ catalyst, an activated $AlF_3$ catalyst and a process for isomerising hydrochlorofluorocarbons.

The invention relates in particular to a process for isomerising 1,1,2-trifluoro-1,2-dichloroethane ($CF_2Cl$-CHF-Cl, hereinafter referred to as HCFC-123a or "asymmetrical" isomer) to 1,1,1-trifluoro-2,2-dichloroethane ($CF_3$-CHCl$_2$, hereinafter HCFC-123 or "symmetrical" isomer), making use of the activated catalyst. More particularly, it relates to a process for obtaining HCFC-123 with a very low content (less than 0.1%, or 1000 ppm, preferably less than 0.05%, or 500 ppm) of the asymmetrical isomer, HCFC-123a, by isomerisation of the HCFC-123a contained in 123/123a mixtures.

The need for having available an industrial process for preparing HCFC-123 as free as possible from the 123a isomer is well known (see e.g. USP 5,600,037).

However, in the most common industrial process for the preparation of HCFC-123, that is, the fluorination of perchloroethylene with HF in the presence of an appropriate catalyst, the product always contains the 123a isomer, in amounts ranging from some thousands of ppm to some parts % : see ex. WO 95/32168, EP Appl. 609123 (also the 123b isomer $CF_2H$-CFCl$_2$ is produced, but in such low amounts that its presence is negligible).

The separation of the isomers with physical methods is exceedingly difficult, due to their similar physico-chemical characteristics (ex. B.P. of 123 = 27.1°C, of 123a = 28.2°C).

Several chemical methods have been proposed, to process the 123/123a mixtures and transform the 123a in another compound, more easily separated from 123 : for example, disproportion to HCFC-124 and HCFC-122 (USP 5.414.167), fluorination to HCFC-124 (USP 5.773.671), dehydrochlorination to CTFE (chlorotrifluoroethylene), etc.

Unfortunately, while in each of these methods the 123a isomer is the most reactive of the two, none of them is selective enough to prevent the loss of substantial amounts of the symmetrical 123 isomer, which reacts along the 123a, albeit at a slower rate. Moreover, even if 100% selectivity could be achieved, the 123a would be in any case converted to some other compound and is lost.

Several methods have thus been proposed for the catalytic isomerisation of 123a to 123, either in the liquid or in the gas phase.

The liquid-phase, homogeneously catalyzed processes are often quite efficient in the isomerisation reaction but suffer several drawbacks, among which the most serious is, as a rule, the low selectivity due to the formation of several undesirable by-products (see e.g. Jap. Appl. 63-85175 of Oct. 16, 1989); a more selective liquid-phase process is disclosed in USP 5,302,766 but it has a very low efficiency: the best result reported is 0.63% residual 123a with a contact time of several hours; more in general, the homogeneous, liquid-phase processes require a more complex and expensive workup of the reaction products than the gas-phase, heterogeneously catalyzed ones.

USP 5,600,037 discloses such a heterogeneous process, in which HCFC-123a is isomerized in the gas phase, on an $AlF_3$ (aluminium fluoride) solid catalyst. However, in the cited patent the aforementioned goal of less than 0.1% of 123a is never obtained.

Indeed, to obtain a product containing less than 0.1% of 123a, it is necessary to run the isomerisation reaction at a temperature lower than those of the examples of the '037 patent; this is due to the fact that conversion of 123a to 123 is equilibrium-limited and the equilibrium constant between the isomers favours the 123 at low temperatures, the 123a at higher ones. See e.g. USP 5.302.766 (to DuPont), whose results have been confirmed by the data obtained by the Applicant.

Thus, to obtain a product containing a low residual amount of 123a, it is necessary to run the isomerisation reaction at a low temperature; in this case, however, the catalyst deactivates quickly and the useful time on stream is unacceptably short : after a few hours the conversion of 123a to 123 decreases and the residual 123a in the exit stream increases far beyond the desired limit: this is disclosed, e.g., in USP 5,118,887, where in the best mode the conversion of 123a decreases from 99.9 to 85% after a mere three hours on stream; the Applicant's own work confirms these data.

Conversely, at higher temperatures the deactivation is slower but the residual 123a cannot be lower than the equilibrium limit: for example, at 350°C the deactivation is negligible but the residual 123a cannot be lower than about 0.3%, due to the isomers' equilibrium.

It has now been surprisingly found by the Applicant that these problems can be overcome by using a catalyst based on aluminium trifluoride ($AlF_3$), which is treated previously to the use in the manner hereinbelow described.

Consequently the invention concerns a process for producing an activated $AlF_3$ based catalyst, wherein a crude $AlF_3$ is treated for more than 5 hours with a gas stream at a temperature from 300°C to 450°C.

When the catalyst obtainable by this process is used, for example, in the gas-phase isomerisation reaction of hydrochlorofluorocarbons such as HCFC-123a, the desactivation of the catalyst is very slow and the reactor needs to be regenerated only at long intervals. The activity of the activated $AlF_3$ catalyst generally remains at its initial level for at least 10 hours. Often the activity can be preserved for at least 50 hours. In a preferred embodiment, said activity is preserved for at least 100 hours. In a particularly preferred embodiment, said activity is preserved for more than 200 hours.

Excellent results are obtained for the isomerisation of hydrochlorocarbons, in particular of HCFC-123a contained in HCFC-123. Isomeric purity of the product after treatment in the presence of the activated $AlF_3$ catalyst is generally more than 99.9% mole. Often a purity equal to or greater than 99.95 mole % is achieved.

In the present description, "aluminium trifluoride ($AlF_3$)" is intended to denote in particular a crystalline solid of such formula, generally obtained from the exhaustive fluorination of aluminium (hydr)oxide (commonly referred to as alumina) with anhydrous hydrogen fluoride (HF), as described e.g. in US 6.187.280 and 6.432.362. During this manufacture of aluminium fluoride, the partially fluorinated aluminas become more and more impervious to further reaction. Consequently, the stoichiometric formula "$AlF_3$" cannot normally be reached by these methods.

The crude $AlF_3$ used in the process according to the invention contains generally at least 90 wt. % of stochiometric $AlF_3$. Preferably, the content of stochiometric $AlF_3$ is at least 95 wt. %. More preferably the content of stochiometric $AlF_3$ is at least 96 wt. %. The crude $AlF_3$ used in the process according to the invention contains generally at most 99.9 wt.% of stochiometric $AlF_3$. Often, the content of stochiometric $AlF_3$ is at most 99 wt. %.

The crude $AlF_3$ used in the process according to the invention has generally a B.E.T. surface area determined by $N_2$ adsorption equal to or greater than 15 $m^2/g$. Often, this specific surface area equal is to or greater than 20 $m^2/g$. A specific surface area equal to or greater than 25 $m^2/g$ is more particularly preferred. The crude $AlF_3$ used in the process according to the invention has generally a B.E.T. surface area less than 100 $m^2/g$. Often, this specific surface area is equal to or less than 75 $m^2/g$. A specific surface area equal to or less than 50 $m^2/g$ is more particularly preferred.

The crude $AlF_3$ is preferably chiefly composed by the crystalline phase. The content of crystalline phase as determined by X-ray diffraction and comparison of the relative peak intensity is generally at least 60%. Preferably, this content is at least 70%. The content of crystalline phase as determined by X-ray diffraction and comparison of the relative peak intensity is generally less than 100%. Preferably, this content is at most 85%.

When the crude $AlF_3$ is obtained by fluorination of alumina, the alumina used as a starting material is preferably in the form of the hydrated aluminium oxide known as boehmite and may optionally contain a minor component of silicon oxide (silica). Such aluminas or silico-aluminas are commercial products, for example the Pural® and Siral® grades of the Sasol (ex Condea) firm.

Moreover, if the catalyst is to be used in a fluid-bed reactor, the alumina has a particle size distribution compatible with this use, as it is well known by the expert in the art.

Beyond the cited patents, both $AlF_3$ as such and the method for its preparation are well known to the art, see e.g. FR 1.383.927.

In the process according to the invention, the temperature of the treatment is preferably equal to or greater than 330°C. A temperature equal to or greater than 350°C is more particularly preferred. In the process according to the invention, the temperature of the treatment is preferably equal to or less than 420°C. A temperature equal to or less than 400°C is more particularly preferred.

In the process according to the invention, the duration of the treatment is preferably equal to or greater than 6 hours. A duration equal to or greater than 10 hours is more particularly preferred. In the process according to the invention, the duration of the treatment is generally equal to or less than 50 hours. A duration equal to or less than 20 hours is more particularly preferred.

In the process according to the invention, the pressure of the treatment is generally equal to or greater than about 1 bar. Atmospheric pressure (about 1 bar) is preferred. In the process according to the invention, the pressure of the treatment is generally equal to or less than about 10 bar. A pressure equal to or less than about 5 bar is preferred.

In the process according to the invention, the contact time of the treatment defined as the ratio between the volume of the reactor and the flow of treatment gas is generally equal to or greater than about 10s. A contact time equal to or greater than about 30s is preferred. In the process according to the invention, the contact time is generally equal to or less than about 100s. A contact time equal to or less than about 50s is preferred.

In the process according to the invention, the gas stream contains often at least one gas selected from air, oxygen, hydrogen fluoride, halogenated hydrocarbon or inert gases. Among the inert gases, particular mention may be made of nitrogen and noble gases such as helium and argon. Among the halogenated hydrocarbons, particular mention may be made of chlorohydrocarbons, fluorohydrocarbons and hydrochlorofluorocarbons. Among the latter, 1,1,1-trifluoro-2,2-dichloroethane and its mixtures with the isomer 1,1,2-trifluoro-1,2-dichloroethane is particularly preferred.

If a halogenated hydrocarbon stream is used, it can consist essentially of the halogenated hydrocarbon or it can optionally be diluted, for example with an inert gas.

If the activated $AlF_3$ catalyst is intended for use for the isomerisation of a hydrochlorofluorocarbon, the activation comprises, in a preferred embodiment, an activation treatment with the hydrochlorofluorocarbon mixture to be isomerised.

In a particularly advantageous embodiment of the process according to the invention, the treatment with the gas stream comprises at least 2 treatment steps with different gases, preferably selected from those mentioned above.

In this embodiment, the treatment with the gas stream comprises preferably at least a treatment step carried out with an anhydrous hydrogen fluoride stream. In this case, the duration of the treatment with the anhydrous hydrogen fluoride stream is generally equal to or greater than 4 hours. Preferably the duration of this treatment is equal to or greater than 6 hours. In this case, the duration of the treatment with the anhydrous hydrogen fluoride stream is generally equal to or less than 16 hours. Preferably the duration of this treatment is equal to or less than 12 hours.

In a particularly preferred embodiment, the temperature of the treatment with anhydrous hydrogen fluoride is about 350°C $\pm$ 25°C or 360°C $\pm$ 25°C.

In a first particularly preferred mode of carrying out the said embodiment, the treatment with the gas stream comprises

(a) a treatment with an inert gas stream for at least 2 hours
(b) optionally, a treatment with an anhydrous hydrogen fluoride stream, in particular as described herebefore,
(c) a treatment with a hydrochlorofluorocarbon containing stream for more than 3 hours.

In this mode, the duration of treatment step (a) is preferably at least 4 hours. The duration of treatment step (a) is generally at most 12 hours. The duration of treatment step (a) is preferably at most 8 hours.

In this mode, the duration of treatment step (c) is preferably at least 7 hours. The duration of treatment step (c) is generally at most 50 hours. The duration of treatment step (c) is preferably at most 25 hours. It should be understood that in this mode the exit gases can be monitored via GC, and the treatment is deemed complete when a constant or declining conversion of hydrochlorofluorocarbon is attained. The time necessary to complete this part of the activation is considerably shortened, if it is preceded by the HF treatment. In a particularly preferred embodiment, the temperature in treatment step (c) is about 350°C $\pm$ 25°C.

In a second particularly preferred mode of carrying out the said embodiment, the treatment with the gas stream comprises

(a) a treatment with an air stream for at least 2 hours
(b) a treatment with an anhydrous hydrogen fluoride stream for at least 4 hours.

In this mode, the duration of treatment step (a) is preferably at least 4 hours. The duration of treatment step (a) is generally at most 12 hours. The duration of treatment step (a) is preferably at most 8 hours.

In a particularly preferred embodiment of this mode, the temperature in treatment step (a) is about 390°C $\pm$ 25°C.

In this mode, the duration of treatment step (b) with anhydrous hydrogen fluoride is as described above.

It is understood that the information of the general description of the process applies also to each of the particular embodiments described herebefore.

The invention concerns also an activated AlF$_3$ catalyst, obtainable according to the process according to the invention.

The activated catalyst obtained according to the process according to the invention may be either unloaded and stored, or immediately used in the same reactor e.g. for the isomerisation of hydrochlorofluorocarbons such as HCFC-123a.

In this case the temperature is suitably adjusted to the value required to obtain the desired content of isomer.

The invention concerns also a process for the isomerisation of a hydrochlorofluorocarbon, wherein the hydrochlorofluorocarbon is contacted with the activated catalyst according to the invention.

In the isomerisation process according to the invention, the hydrochlorofluorocarbon is preferably in the vapor state.

The isomerisation process according to the invention can be carried out in a fixed bed reactor or in a fluidized bed reactor. A fluidized bed reactor is preferred.

Hydrofluorocarbons which can suitably be isomerised by the isomerisation process according to the invention include, amongst others, 1,1,2-trifluoro-1,2,-dichloroethane, 1,1,2-trifluoro-1 -chloroethane, 1,2-difluoro-1-chloroethane, 1, fluoro-1,2,-dichloroethane, 1,1,3-trifluoro-1,3,-dichloropropane and 1,1,3,3-tetrafluoro-3-chloropropane. The isomerisation of 1,1,2-trifluoro-1,2,-dichloroethane is particularly preferred. The following description is specifically drawn to the isomerisation reaction of 1,1,2-trifluoro-1,2,-dichloroethane, it should however be understood that the subject or similar conditions apply also to other hydrochlorofluorocarbons.

In a preferred embodiment of the isomerisation process according to the invention, the hydrochlorofluorocarbon introduced into the reactor comprises a mixture of 1,1,1-trifluoro-2,2,-dichloroethane (123) and 1,1,2-trifluoro-1,2,-dichloroethane (123a).

In this embodiment, the 123/123a mixture fed to the reactor contains generally at least 0.3 wt. % of 123a with reference to the total weight of the 123/123a mixture. Often this content is equal to or greater than about 0.5 wt. %.

More frequently, this content is equal to or greater than about 1 wt. %. In this embodiment, the 123/123a mixture fed to the reactor contains generally at most 50 wt. % of 123a with reference to the total weight of the 123/123a mixture. Often this content is equal to or less than about 20 wt. %. More frequently this content is equal to or less than about 15 wt. %.

In this embodiment, the organic feed introduced into the isomerisation reaction may contain, beside the 123 isomers, also substantial amounts of different compounds, such as those found as by-products in a 123 production process such as contemplated herebefore. It has been found that these compounds do not adversely affect the isomerisation process according to the invention. The organic feed introduced into the isomerisation reaction may typically contain up to approx. 20% by weight of by-products and 80% weight of 123 isomers.

Surprisingly, no appreciable difference in the results of the isomerisation reaction has been found, whichever the isomeric composition of the organic feed. The isomerisation process according to the invention is flexible enough to treat, for example, all possible isomeric compositions of an HCFC-123 produced e.g. via fluorination of perchloroethylene, under industrially relevant conditions. If a sufficient conversion of 123a to 123 is not reached because of a very high content of 123a in the feed, this can be overcome by adjusting the recycle loop of the reactor until the appropriate inlet composition has been achieved.

In the isomerisation process according to the invention, the organic feed may optionally be diluted in an inert gas, such as described above. If present, the inert gas fraction of the total stream is generally at least 5% by volume. In this case, this fraction is preferably at least 10% by volume. If present, the inert gas fraction of the total stream is generally at most 90% by volume. In this case, this fraction is preferably at most 50% by volume.

In the isomerisation process according to the invention, the temperature is preferably equal to or greater than 150°C. A temperature equal to or greater than 180°C is more particularly preferred. A temperature about equal to or greater than 200°C is most particularly preferred. In the isomerisation process according to the invention, the temperature is preferably equal to or less than 260°C. A temperature equal to or less than 230°C is more particularly preferred. A temperature about equal to or less than 220°C is most particularly preferred.

The pressure of the reactor does not adversely affect the isomerisation reaction, provided it is not so high to cause the condensation of the hydrochlorofluorocarbon at the reaction temperature.

In the isomerisation process according to the invention, the pressure is generally equal to or greater than about 1 bar. A pressure equal to or greater than about 2 bar is preferred. In the isomerisation process according to the invention, the pressure is generally equal to or less than about 10 bar. A pressure equal to or less than about 5 bar is preferred.

While the isomerisation of 123a is not adversely affected by the pressure of the reactor, it has been found, surprisingly, that a moderately high pressure has a beneficial effect on the formation of unsaturated by-products. In particular, when the process is run at a pressure which is moderately higher than the atmospheric, preferably at 2 to 5 bar abs., the formation of the unsaturated by-product CFC-1112a (1,1-dichloro-2,2-difluoroethylene, $CF_2=CCl_2$) is greatly reduced or even eliminated altogether. This is desirable since CFC-1112a is both toxic and difficult to separate from HCFC-123.

The invention concerns in consequence also a method for the isomerisation of 1,1,2-trifluoro-1,2,-dichloroethane wherein the 1,1,2-trifluoro-1,2,-dichloroethane, preferably in the vapor state, is contacted with an isomerisation catalyst under a pressure of from 2 to 5 bar.

The particular embodiments of the method according to the invention correspond to those of the isomerisation process according to the invention.

The instant invention is illustrated in a non-limitative manner by the following examples:

Example 1 - Activation of the catalyst

A sample of 9.0 g of aluminium trifluoride in form of a fine powder obtained by fluorination of a SIRAL® 1.5 silico-alumina using the general fluorination procedure described in US 6.187.280 (examples) was loaded into a tubular reactor of 3/8 inches o.d. (ca 15 mm internal diameter), heated by means of an electric oven, and brought to the temperature of 350°C in an helium flow.

After 4 hours at 350°C, a flow of 2.1 scc (cubic centmeters at 20°C and atmospheric pressure)/min (0.8 g/h) of HCFC-123, containing 11.9% 123a, was added to the feed. The pressure in the reactor was atmospheric, the total flow of He and organics was 7 scc/min.

The gas exiting from the reactor was sampled an analysed via GLC; the flow was monitored at about 40min intervals, witnessing an increase of the conversion of 123, which reached a maximum after ca. 9 hrs on stream.

Both isomers 123 were converted to several disproportionation products: the following analysis, at 9 hrs on stream, is typical of the composition of the products when the maximum conversion had been attained:

| HCFC-124* | 9.7% weight |
|---|---|
| HCFC-133a | 1.7 |
| CFC-114* | 3.6 |
| CFC-113* | 0.6 |
| HCFC-123a | 0.3 |
| HCFC-123 | 76.8 |
| CFC-1112* | 0.1 |
| HCFC-1111* | 5.3 |
| HCFC-122* | 1.0 |
| others | 0.9 |

*Mixture of isomers

The residual 123a/123 content was stable at ca. 0.35%, which is the about equilibrium limit at 350°C.
When the conversion of 123 had reached the maximum, the temperature of the reactor is lowered to the desired isomerisation temperature.

Example 2 - Isomerisation of 123a at 220°C (fixed bed reactor)

Continuing from ex. 1, the temperature of the reactor was lowered to 220°C and the sampling was resumed. The following analysis of the obtained products shows that at this temperature the formations of by-products was negligible and the residual 123a/123 content was down to ca. 500 ppm

| HCFC-123a | 0.05 |
|---|---|
| HCFC-123 | 99.1 |
| CFC-1112a | n.d. |
| others | 0.85 |

The selectivity (measured as the fraction of 123 recovered) was equal to 99.2%; the above results were obtained consistently during the whole run (about 6 hours), with no deactivation of the catalyst.

Example 3 - Isomerisation of 123a at 200°C

After an interval, the testing was resumed with the same catalyst used in Examples. 1 and 2, which was briefly reactivated with a flow of He/123 at 350°C, then the temperature of the reactor was lowered to 200°C. The same stream of ex. 2 was fed to the reactor at atmospheric pressure.
The products were sampled during a 4 hrs period. Their composition was substantially stable throughout the run, with the following analytical result:

| HCFC-123a | 0.04 |
|---|---|
| HCFC-123 | 99.5 |
| CFC-1112a | n.d. |
| others | 0.45 |

The residual 123a/123 was about 400 ppm, lower than in ex. 2, and the selectivity as well was increased, to about 99.5%.

Example 4 - Isomerisation of 123a at 200°C with an HCFC-123a containing a lower amount of HCFC-123a

Example 3 was reproduced (with a fresh catalyst sample, previously activated as in Ex. 1) with the only change that the HCFC-123 mix contained 3.8% wt. of the asymmetrical isomer; no meaningful difference in the results was detected, as results from the following analysis of the products:

| HCFC-123a | 0.04 |
|---|---|

(continued)

| HCFC-123 | 99.6 |
|---|---|
| CFC-1112a | n.d. |
| others | 0.035 |

The residual 123a content in the products was extremely low and the overall selectivity of the reaction was the same as in Ex. 3.

Example 5 - Comparison with Example 4

A fresh sample of the same AlF$_3$ used in Ex. 1-4 has been used in comparison test: thus, 10g of catalyst were loaded in the same reactor and dehydrated at 350°C in an He flow for only 1 hr. then activated with an HCFC-123 flow at 350°C in the same conditions as in Ex. 4 but for two hours only.

The temperature was then lowered to 210°C and the same He/123 flow of Ex. 4 (with 3.8% 123a) was fed to the reactor.

The conversion of 123a decreased fast and after 5 hrs on stream the content in the product gas had reached 0.26%, far higher than the desired value.

Thus omitting the activation according to the invention resulted also in an unacceptably short useful life of the catalyst.

Example 6 - Fixed bed reaction with pelletized catalyst

In this example a pelletized catalyst was employed. This catalyst is especially suitable for the use in industrial-scale fixed-bed reactors.

400 g (375 cc) of AlF$_3$ pellets were loaded in a tubular reactor, 50 mm i.d., equipped with a fritted bottom and thermally insulated. In this setup, the reactor was operated in so-called "adiabatic" mode, where the heat is furnished to the reaction feed in an exchanger before entering the reactor.

The pressure in the reactor was atmospheric both during the activation and the isomerisation test.

The reactor was brought to 360°C in a nitrogen flow, then the catalyst was first treated in an HF flow, purged from HF again in a nitrogen flow and finally activated with a 123 flow for a period of 20 hrs, in the following conditions :

Temperature 350°C, 123 feed 0.266 mol/h (41 g/h) diluted with 21 sL/h of nitrogen; the nominal contact time at 350°C and atm. pressure was 33 s; the HCFC-123 contained 0.3% (3000 ppm) of the asymmetric isomer.

The reactor temperature was then adjusted to 240°C while the same flow was fed to the reactor, thus realizing a contact time of 40 s.

The reaction products were sampled and analyzed and a residual 123a content of 0.025 (250 ppm) was found; this result remained constant for the whole duration of the test, 30 h on stream.

Example 7 - Isomerisation of 123a in a fluid-bed reactor

400 g (380 cc) of fresh AlF$_3$, with a particle size distribution suitable for the use as a fluid-bed catalyst, were loaded in a tubular reactor of 50 mm equipped with a fritted bottom and heated by means of electric resistors. The catalyst was heated up to 390°C in a nitrogen flow, then treated for 4 hr with an air flow at 390°C. Then the temperature of the reactor was set to 360°C, the air flow was discontinued and replaced by an HF flow. After 10 hrs the HF flow was stopped and the reactor was purged in nitrogen flow for a few hrs, while the temperature was gradually lowered to 240°C.

When the reactor temperature had stabilized at 240°C, a reaction mixture of the following composition was fed to the reactor : 40.7 g/h (0.266 mol/h) of HCFC-123 (containing 0.3% HCFC-123a) and ca. 15 NL/h (0.663 mol/h) of nitrogen.

The nominal contact time was 35 s at the reaction temperature and atmospheric pressure.

The products were sampled and analyzed via GC with the following result:

| HCFC-124+124a | 1.61 mol % |
|---|---|
| HCFC-123a | 0.067 |
| HCFC-123 | 97.47 |
| CFC-1112a | 0.33 |

**EP 1 502 906 A1**

(continued)

| others | 0.52 |
|---|---|

The catalytic activity remained stable during the whole run (20 hrs), with no sign of deactivation. The residual 123a content remained on average at about 700 ppm and the selectivity around 97.5%.

Example 8

The test of ex. 7 was continued at a lower temperature, without any other alteration. The temperature of the reactor was set at 210°C without even stopping the reactant flow. The flow was not adjusted to the new temperature, thus the contact time became slightly higher than in. ex. 7.
The residual 123a dropped to 400 ppm and the conversion of 124 and formation of 1112 dropped too:

| HCFC-124+124a | 0.62 mol % |
|---|---|
| HCFC-123a | 0.034 |
| HCFC-123 | 98.99 |
| CFC-1112a | 0.14 |
| others | 0.22 |

Both the selectivity (about 99%) and the residual content of 123a isomer about 350-400 ppm remained substantially constant for the duration of the test, 70 hrs on stream.

Example 9

Example 7 was repeated with a fresh catalyst sample, which was activated in the same manner; at a temperature of 240°C, a flow of HCFC-123 was fed to the reactor under identical conditions as in example 7, excepted that the 123a content in the feed stream was 1.4% wt. The residual 123a in HCFC-123 was stable at about 700 ppm.

| HCFC-124+124a | 2,23 mol % |
|---|---|
| HCFC-123a | 0.063 |
| HCFC-123 | 96.26 |
| CFC-1112a | 0.55 |
| others | 0.90 |

Example 10

In an analogous manner as described in examples 7 and 8, the reactor temperature was lowered to 210°C at the end of the procedure of example 9 without stopping the reactant flow. The residual 123a dropped to about 400 ppm and the content of by-products as well.

| HCFC-124+124a | 0.57 mol % |
|---|---|
| HCFC-123a | 0.038 |
| HCFC-123 | 99.14 |
| CFC-1112a | 0.10 |
| others | 0.15 |

The catalyst showed no deactivation for the whole duration of Example 10, ca 35 hrs on stream

Example 11 - Isomerisation of 123a at superatmospheric pressure in a fluid-bed reactor

In the same experimental setup of example 7, 400 g of fresh $AlF_3$ was loaded and activated in the same manner, as in that example.
After the activation, the temperature was set at 240°C and the reactor pressure at 3 atm. absolute (2 atm. above atmospheric), and a mixture of 40.7 g/h (0.266 mol/h) of HCFC-123 (containing 0.3% 123a) and ca. 63 NL/h of nitrogen, was supplied to the reactor.

The following results were obtained:

| | |
|---|---|
| HCFC-124 | 1.37 mol % |
| HCFC-123a | 0.062 |
| HCFC-123 | 98.42 |
| CFC-1112a | 0.20 |
| others | 0.05 |

These results remained constant for 160 hrs on stream, during which the catalyst showed no sign of deactivation.

It is readily apparent that the pressure increase had no adverse effect on the isomerisation of 123a, while it is quite effective in reducing the by-production of 1112a, which is a toxic and undesirable compound.

Example 12

The test of example 1 was continued without regeneration of the catalyst; the total feed streams remained the same but the 123a content in HCFC-123 was increased to 1.5% wt. with the following result:

| | |
|---|---|
| HCFC-124 | 0.68 mol % |
| HCFC-123a | 0.055 |
| HCFC-123 | 98.90 |
| CFC-1112a | 0.25 |
| others | 0.12 |

This example shows that when using a 123 feed with an higher content of 123a, the results were not affected and the residual 123a in the products reached still the equilibrium limit. These results remained constant up to the end of the test i.e. more than 200 hrs on stream from the beginning of example 1. A decrease in time of the side reaction to HCFC-124 has also been observed.

Example 13

The catalyst used in Examples 1 and 12 was regenerated by means of an air treatment at 390°C for 4 hours followed by fluorination with HF at 360°C for 12 hours.

After the regeneration the 123/123a (ca. 4000 ppm 123a) flow was restored with the same pressure and feedrates as in the examples 1 and 12, while the reaction temperature was set at 220°C. At this lower temperature, the residual 123a in 123 decreased to 400 ppm average and remained stable for the whole duration of the test, 140 hrs. The 1112a and other byproducts decreased as well:

| | |
|---|---|
| HCFC-124 | 0.58 mol % |
| HCFC-123a | 0.035 |
| HCFC-123 | 99.29 |
| CFC-1112a | 0.06 |
| others | 0.04 |

Example 14

The catalyst used in examples 11-13 was regenerated as above described and the pressure in the reactor was set to 5 ata (4 bar gauge). The temperature was 220°C.

To conserve the same volumetric contact time, both the 123 and nitrogen feedrates were increased to:

$$123 = 0.443 \text{ mol/h} = 68 \text{ g/h}$$

nitrogen = 104 sL(liter at 20°C and atmospheric pressure)/h

The following result was obtained:

| HCFC-124 | 0.05 |
|---|---|
| HCFC-123a | 0.06 |
| HCFC-123 | 99.89 |

Any other byproduct was lower than the analytical threshold. Thus the pressure is shown to have a very beneficial effect indeed.

## Claims

1. Process for producing an activated $AlF_3$ based catalyst, wherein a crude $AlF_3$ is treated for more than 5 hours with a gas stream at a temperature from 300°C to 450°C.

2. Process according to claim 1, wherein the temperature is from 350°C to 400°C.

3. Process according to claim 1 or 2, wherein the crude $AlF_3$ is treated with the gas stream for from 6 to 50 hours.

4. Process according to any one of claims 1 to 3, wherein the crude $AlF_3$ contains at least 95 wt.% of stochiometric $AlF_3$.

5. Process according to any one of claims 1 to 4, wherein the crude $AlF_3$ has a B.E.T surface of at least 25 $m^2/g$.

6. Process according to any one of claims 1 to 5, wherein the gas stream contains at least one of air, hydrogen fluoride, halogenated hydrocarbon or inert gas.

7. Process according to any one of claims 1 to 6, wherein the treatment with the gas stream comprises at least 2 treatment steps with different gases.

8. Process according to claim 7, wherein the treatment with the gas stream comprises

   (a) a treatment with an inert gas stream for at least 4 hours
   (b) optionally, a treatment with an anhydrous hydrogen fluoride stream
   (c) a treatment with a hydrochlorofluorocarbon-containing stream for more than 1 hour.

9. Process according to claim 7, wherein the treatment with the gas stream comprises

   (a) a treatment with an air stream for at least 2 hours
   (b) a treatment with an anhydrous hydrogen fluoride stream for at least 4 hours.

10. Activated $AlF_3$ catalyst, obtainable according to the process of any one of claims 1 to 9.

11. Process for the isomerisation of a hydrochlorofluorocarbon, wherein the hydrochlorofluorocarbon is contacted with the catalyst according to claim 10.

12. Process according to claim 11 wherein the hydrochlorofluorocarbon is in the vapor state.

13. Process according to claim 11 or 12, wherein the hydrochlorofluorocarbon comprises a mixture of 1,1,1-trifluoro-2,2-dichloroethane and 1,1,2-trifluoro-1,2-dichloroethane.

14. Process according to claim 13 wherein the isomerisation is carried out at a temperature of 180 to 220°C.

15. Method for the isomerisation of 1,1,2-trifluoro-1,2-dichloroethane wherein the 1,1,2-trifluoro-1,2-dichloroethane, preferably in the vapor state, is contacted with an isomerisation catalyst under a pressure of from 2 to 5 bar.

**European Patent Office**

## EUROPEAN SEARCH REPORT

**Application Number**

EP 03 10 2385

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 881 201 A (AUSIMONT SPA) 2 December 1998 (1998-12-02) * page 2, line 53 - line 58 * * claims 1,4; examples 1A,1B * --- | 1-4,6-8, 10 | C07C17/358 |
| X | US 3 787 331 A (CASTELLAN A ET AL) 22 January 1974 (1974-01-22) * column 4, line 39 - line 49; examples 2,5; table 4 * --- | 1-3,6-8, 10-12 | |
| X | US 5 731 481 A (LANTZ ANDRE ET AL) 24 March 1998 (1998-03-24) * examples 1A,1B,2A,2B * --- | 1-8,10 | |
| X | US 5 616 820 A (CHEMINAL BERNARD ET AL) 1 April 1997 (1997-04-01) * column 6, line 8 - line 35; examples A,B; table 1 * --- | 1-3,6-8, 10-13 | |
| X,D | US 5 600 037 A (CUZZATO PAOLO ET AL) 4 February 1997 (1997-02-04) * example 3; table 3 * ----- | 1-3,7, 9-13 | TECHNICAL FIELDS SEARCHED (Int.Cl.7) C07C |

The present search report has been drawn up for all claims

| Place of search | Date of completion of the search | Examiner |
|---|---|---|
| MUNICH | 26 November 2003 | Holzwarth, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 502 906 A1**

### ANNEX TO THE EUROPEAN SEARCH REPORT
### ON EUROPEAN PATENT APPLICATION NO.

EP 03 10 2385

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2003

| Patent document cited in search report | | Publication date | Patent family member(s) | | Publication date |
|---|---|---|---|---|---|
| EP 0881201 | A | 02-12-1998 | IT | MI971237 A1 | 27-11-1998 |
| | | | DE | 69807980 D1 | 24-10-2002 |
| | | | DE | 69807980 T2 | 31-07-2003 |
| | | | EP | 0881201 A1 | 02-12-1998 |
| | | | JP | 10330298 A | 15-12-1998 |
| | | | US | 6300529 B1 | 09-10-2001 |
| US 3787331 | A | 22-01-1974 | DK | 138673 B | 16-10-1978 |
| | | | AT | 292641 B | 10-09-1971 |
| | | | BE | 740090 A | 10-04-1970 |
| | | | CH | 525710 A | 31-07-1972 |
| | | | DE | 1950804 A1 | 29-10-1970 |
| | | | ES | 372331 A1 | 16-02-1972 |
| | | | FR | 2020296 A5 | 10-07-1970 |
| | | | GB | 1283386 A | 26-07-1972 |
| | | | IL | 33144 A | 29-06-1973 |
| | | | JP | 55012297 B | 01-04-1980 |
| | | | NL | 6914962 A ,B, | 14-04-1970 |
| | | | NO | 125479 B | 18-09-1972 |
| | | | SE | 361135 B | 22-10-1973 |
| | | | YU | 254669 A ,B | 28-02-1975 |
| | | | ZA | 6907069 A | 27-01-1971 |
| US 5731481 | A | 24-03-1998 | FR | 2669022 A1 | 15-05-1992 |
| | | | AT | 115532 T | 15-12-1994 |
| | | | AU | 641292 B2 | 16-09-1993 |
| | | | AU | 8779991 A | 14-05-1992 |
| | | | CA | 2055281 A1 | 14-05-1992 |
| | | | DE | 69105924 D1 | 26-01-1995 |
| | | | DE | 69105924 T2 | 27-07-1995 |
| | | | EP | 0486333 A1 | 20-05-1992 |
| | | | ES | 2066394 T3 | 01-03-1995 |
| | | | FI | 915341 A | 14-05-1992 |
| | | | JP | 2053012 C | 10-05-1996 |
| | | | JP | 4288027 A | 13-10-1992 |
| | | | JP | 7080796 B | 30-08-1995 |
| | | | KR | 9506521 B1 | 16-06-1995 |
| | | | NO | 914107 A ,B, | 14-05-1992 |
| | | | PT | 99489 A | 30-09-1992 |
| | | | DK | 486333 T3 | 01-05-1995 |
| | | | GR | 3015222 T3 | 31-05-1995 |
| | | | IE | 913932 A1 | 20-05-1992 |
| | | | MX | 9102049 A1 | 08-07-1992 |
| US 5616820 | A | 01-04-1997 | FR | 2700770 A1 | 29-07-1994 |
| | | | AU | 663816 B2 | 19-10-1995 |

EPO FORM P0459

For more details about this annex : see Official Journal of the European Patent Office, No. 12/82

13

## ANNEX TO THE EUROPEAN SEARCH REPORT
## ON EUROPEAN PATENT APPLICATION NO.

EP 03 10 2385

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2003

| Patent document cited in search report | | Publication date | Patent family member(s) | | Publication date |
|---|---|---|---|---|---|
| US 5616820 | A | | AU | 5395194 A | 18-08-1994 |
| | | | BR | 9400338 A | 16-08-1994 |
| | | | CA | 2113511 A1 | 28-07-1994 |
| | | | CN | 1101337 A ,B | 12-04-1995 |
| | | | DE | 69400342 D1 | 05-09-1996 |
| | | | DE | 69400342 T2 | 23-01-1997 |
| | | | EP | 0609124 A1 | 03-08-1994 |
| | | | ES | 2091097 T3 | 16-10-1996 |
| | | | GR | 3021203 T3 | 31-12-1996 |
| | | | JP | 2510126 B2 | 26-06-1996 |
| | | | JP | 6247883 A | 06-09-1994 |
| | | | KR | 9700398 B1 | 09-01-1997 |
| US 5600037 | A | 04-02-1997 | IT | 1251957 B | 27-05-1995 |
| | | | AT | 151403 T | 15-04-1997 |
| | | | AU | 652080 B2 | 11-08-1994 |
| | | | AU | 2703892 A | 22-04-1993 |
| | | | BR | 9203978 A | 27-04-1993 |
| | | | CA | 2080894 A1 | 19-04-1993 |
| | | | CZ | 9203110 A3 | 16-06-1993 |
| | | | DE | 69218870 D1 | 15-05-1997 |
| | | | DE | 69218870 T2 | 06-11-1997 |
| | | | EP | 0537759 A2 | 21-04-1993 |
| | | | ES | 2103021 T3 | 16-08-1997 |
| | | | GR | 3023176 T3 | 30-07-1997 |
| | | | JP | 3231431 B2 | 19-11-2001 |
| | | | JP | 6128180 A | 10-05-1994 |
| | | | KR | 236863 B1 | 15-01-2000 |
| | | | RU | 2089534 C1 | 10-09-1997 |
| | | | ZA | 9207873 A | 27-04-1993 |

EPO FORM P0459

For more details about this annex : see Official Journal of the European Patent Office, No. 12/82